# EUROPÄISCHE PATENTSCHRIFT

(11) **EP 0 263 448 B1**
(45) Veröffentlichungstag und Bekanntmachung des Hinweises auf die Patenterteilung: **30.06.1993**
(21) Anmeldenummer: 87114434.1
(22) Anmeldetag: 02.10.1987
(51) Int. Cl.: G01N 33/18

(54) **Verfahren zur Analyse von Wasser sowie Vorrichtung zur Durchführung dieses Verfahrens**
Method for analysing water and apparatus for carrying out the method
Procédé pour l'analyse de l'eau et appareil pour utiliser le procédé

(30) Priorität: 04.10.1986 DE 3633842
(43) Veröffentlichungstag der Anmeldung: 13.04.1988
(73) Patentinhaber: FORSCHUNGSZENTRUM JÜLICH GMBH, 52425 Jülich (DE)
(72) Erfinder: Krumpen, Peter, D-5163 Langerwehe (DE); Landgraf, Britta, D-5170 Jülich-Selgersdorf (DE); Prast, Hartmut, D-5120 Herzogenrath-Hofstadt (DE); Schmitz, Bernd, D-5162 Niederzier 2 (DE)
(74) Vertreter: Paul, Dieter-Alfred, Dipl.-Ing.

(56) Entgegenhaltungen:
- EP-A- 0 201 776
- DE-A- 3 217 987
- CHIMIA, Band 25, Nr. 12, Dezember 1971; W.G.STOLL, Seiten 384-393#

## Beschreibung

Die Erfindung betrifft ein Verfahren zur Analyse von Wasser oder anderen Flüssigkeiten, insbesondere von Niederschlagswasser in Regensammlern, bei dem das Wasser für eine Mengenmessung zunächst in einer Volumenmeßzelle bis zum Erreichen eines bestimmten, immer gleichen Füllstandes gesammelt wird, bevor das so gesammelte Wasser jeweils an eine nachfolgende Eigenschaftsmeßzelle, insbesondere einer Leitfähigkeitsmeßzelle und/oder eine pH-Wert-Meßzelle, weitergegeben wird. Die Erfindung betrifft ferner eine Vorrichtung zur Durchführung des Verfahrens mit einer Mengenmeßeinrichtung und zumindest einer nachfolgenden Eigenschaftsmeßzelle, insbesondere einer Leitfähigkeitsmeßzelle und/oder pH-Wert-Meßzelle, wobei die Mengenmeßeinrichtung eine mit einer Füllstandssonde ausgestattete Volumenmeßzelle aufweist, die derart ansteuerbar ist, daß das Wasser erst bei Erreichen der Füllstandssonde aus der jeweiligen Volumenmeßzelle herausgelassen und zur Eigenschaftsmeßzelle weitergeleitet wird.

Bekannt ist der Niederschlags-Monitor nach System Deutscher Wetterdienst, meteorologisches Observatorium Hamburg. Mit ihm können Eigenschaftsmessungen vorgenommen werden, wie z. B. die Erfassung der elektrischen Leitfähigkeit, des ph-Wertes und der Niederschlagsmenge. Die Messung von ph-Wert und elektrischer Leitfähigkeit erfolgt dabei kontinuierlich. Anschließend wird das Wasser zu einer bistabilen Wippe geführt, mittels der die Niederschlagsmenge und auch deren Intensität gemessen wird. Ein eventueller Niederschlag wird durch einen sogenannten Regensensor erfaßt, der einen Abdeckmechanismus über dem Sammeltrichter öffnet und nach dem Ende des Niederschlags auch wieder schließt. Durch eine einstellbare Abschaltverzögerung wird ein häufiges Öffnen und Schließen zu Beginn und Ende eines Niederschlages vermieden.

Die US-A-4 665 743 zeigt einen Niederschlagssammler mit zwei Sammeleingängen. Allerdings dient nur einer der Eingänge der Sammlung des Niederschlages, während der andere Eingang für die Sammlung von Feststoffen während der Zeit, in der kein Niederschlag erfolgt, dient. Bei diesem vorbekannten Niederschlagssammler mit einer Volumenmeßzelle ist eine genaue Trennung der beiden aufeinanderfolgenden Volumina nicht möglich, und zwar auf Grund der Tatsache, daß während der öffnungszeit des Ventils weiterer Niederschlag in die Volumenmeßzelle einfällt und es deshalb zu Vermischungen zwischen dem gerade über das Ventil abfließenden Volumen mit dem nachfolgenden Niederschlag kommt. Auf diese Weise können Änderungen in den Eigenschaften des Regens nicht exakt ermittelt werden. Für wissenschaftliche Untersuchungen des Niederschlages ist jedoch eine exakte Trennung der zu analysierenden Volumina erforderlich, damit die Änderung der Meßwerte der jeweiligen Zeit exakt zugeordnet werden kann.

In der US-A-4 140̸ 0̸11 ist ein Niederschlagssammler offenbart, bei dem der Niederschlag noch in Behältern gesammelt wird, die zur Analyse des Inhalts abgenommen und in ein Labor transportiert werden müssen. Der Wechsel der Behälter erfolgt nicht volumenabhängig, sondern geschieht zeitgesteuert, d. h. die Umschaltung erfolgt nach einer bestimmten Zeit. Entsprechend enthalten die Behälter verschiedene Volumina, wenn die Regenintensität variiert.

An die Genauigkeit der Messung insbesondere im Anfangsstadium, wenn der Niederschlag besonders schadstoffbelastet ist, werden immer größere Anforderungen gestellt. Außerdem besteht das Bedürfnis, das gesammelte Wasser schon so aufzubereiten, daß eine anschließende Analyse insbesondere auf organische Substanzen und auf Schwermetalle ohne besondere Vorbereitungen möglich ist.

Der Erfindung liegt somit die Aufgabe zugrunde, ein Verfahren sowie eine Vorrichtung zur Durchführung des Verfahrens zu finden, mit dem bzw. mit der eine größere Genauigkeit der Messung insbesondere in der Anfangsphase eines Niederschlages erzielbar ist und das bzw. die eine anschließende Aufbereitung des gesammelten Wassers ermöglicht.

Diese Aufgabe wird erfindungsgemäß dadurch gelöst, daß das Wasser für die Mengenmessung abwechseln in zumindest zwei parallelen Volumenmeßzellen gesammelt und abwechselnd an die nachfolgende Eigenschaftsmeßzelle weitergegeben wird.

Bei diesem Verfahren wird also das Wasser abwechselnd in der einen oder in der anderen Volumenmeßzelle gesammelt, wobei die Umschaltung jeweils dann erfolgt, wenn in einer Volumenmeßzelle der Maximalfüllstand erreicht ist und das so gesammelte Wasser an die anschließende Eigenschaftsmeßzelle weitergegeben wird. Auf diese Weise wird trotz kleiner Volumina in den Volumenmeßzellen hinreichend Zeit für die anschließenden Messungen gewonnen.

Nach einem weiteren Merkmal der Erfindung ist vorgeschlagen, daß das Wasser durch eine Adsorptionssäule zur Entferung organischer Bestandteile geleitet und anschließend gesammelt wird. Die organischen Bestandteile können dann analysiert werden. Das auf diese Weise erhaltene Wasser ist - je nach Art der Adsorptionssäule - von organischen Schadstoffen befreit und kann dann vom Regensammler direkt zur Analyse anorganischer Schadstoffe im Labor, insbesondere von Schwermetallverunreinigungen, benutzt werden.

Dabei ist es zweckmäßig, daß das Wasser vorher in einem Sammelbehälter gesammelt und dann bis auf einen bestimmten Rest durch die Adsorptionssäule geleitet wird, damit die Adsorptionssäule nicht beschädigt wird. Für den Fall, daß die Adsorptionssäule auszutrocknen beginnt, also das Restwasser im Sammelbehälter unter ein bestimmtes Niveau absinkt, ist vorgesehen, die Adsorptionssäule mit destilliertem Wasser zu füllen. Umgekehrt sollte das Wasser erst bei Erreichen eines bestimmten Maximalstandes durch die Adsorptionssäule geleitet werden, zumindest jedoch am Ende jeder Messung. Damit das Wasser durch die Adsorptionssäule mit gleichmäßigem Durchfluß geleitet wird, sollte es dort durchgesaugt werden.

Eine Vorrichtung zur Durchführung des vorbeschriebenen Verfahrens ist erfindungsgemäß dadurch gekennzeichnet, daß wenigstens zwei Volumenmeßzellen vorgesehen sind, die abwechselnd durch eine Steuereinrichtung derart ansteuerbar sind, daß der Zufluß nur zu jeweils einer Volumenmeßzelle geöffnet ist und bei Erreichen der Füllstandssonde in dieser Volumenmeßzelle geschlossen und zu einer anderen Volumenmeßzelle geöffnet wird. Durch diese besonders günstige Ausführungsform wird für die nachfolgenden Messungen Zeit gewonnen, ohne daß das Sammeln des Wassers unterbrochen wird.

In weiterer Ausbildung der Erfindung ist vorgeschlagen, daß eine Adsorptionssäule zur Entfernung und anschließenden Analyse organischer Bestandteile vorgesehen und daran anschließend ein Sammelbehälter nachgeschaltet sind, aus dem dann das Wasser ohne weitere Aufbereitung zur Analyse anorganischer Schadstoffe, insbesondere von Schwermetallverunreinigungen, im Labor genommen werden kann. Zweckmäßigerweise ist vor der Adsorptionssäule ein weiterer Sammelbehälter angeordnet, damit das Wasser zunächst gesammelt werden kann, bevor es durch die Adsorptionssäule geleitet wird. Hierzu sollte der weitere Sammelbehälter mit einem mit der Steuereinrichtung in Verbindung stehenden Füllstandsensor für die Ermittlung des Minimalniveaus ausgestattet und die Adsorptionssäule mit einem Vorratsbehälter für destilliertes Wasser verbunden sein, dessen Zulauf durch die Steuereinrichtung geöffnet wird, wenn der Füllstandsensor ein Unterschreiten des Minimalniveaus meldet. Auf diese Weise wird verhindert, daß die Adsorptionssäule austrocknet und dabei beschädigt wird.

Zusätzlich ist vorgesehen, daß der weitere Sammelbehälter mit einem Füllstandsensor zur Erfassung eines Maximalniveaus ausgestattet ist, der über die Steuereinrichtung mit einer Pumpe zur Förderung des Wassers durch die Adsorptionssäule derart verbunden ist, daß die Pumpe bei Erreichen des Maximalniveaus in Betrieb gesetzt wird. Erfindungsgemäß soll also zunächst Wasser angesammelt werden, damit es dann kontinuierlich durch die Adsorptionssäule gefördert wird. Ansonsten soll die Pumpe auf jeden Fall in Betrieb gesetzt werden, wenn eine Messung beendet ist.

Nach der Erfindung ist ferner vorgesehen, daß zwischen einer Eigenschaftsmeßzelle und dem weiteren Sammelbehälter ein Dreiwegeventil zur Freigabe einer Leitung zu einem Abwasserbehälter vorgesehen ist. Dieses Dreiwegeventil sollte über die Steuereinrichtung derart ansteuerbar sein, daß nur die Meßprobem in den weiteren Sammelbehälter gelangen.

Die erfindungsgemäße Vorrichtung eignet sich besonders als Niederschlagssammler, um den Niederschlag entsprechend analysieren zu können. Hierzu sollte in an sich bekannter Weise ein Regenfühler vorgesehen sein, der Beginn und Ende einer Messung bestimmt. Der Regenfühler sollte mit einem Verzögerungsglied zur Beendigung der Messung in einem zeitlichen Abstand nach Wegfall der Benetzung versehen sein, damit die Messung bei geringem Niederschlag nicht dauernd unterbrochen wird.

In besonders bevorzugter Ausführung weist die Vorrichtung ein herausnehmbares Speicherglied für die Abspeicherung von Daten, insbesondere von Datum und Uhrzeit der jeweils ersten Benetzung des Regenfühlers, von Datum, Uhrzeit und Nummer der Füllung einer Volumenmeßzelle, der Gesamtniederschlagsmenge sowie gegebenenfalls der Leitfähigkeit und des ph-Wertes sowie von Datum und Uhrzeit des Meßendes. Solche Speicherglieder sind an sich bekannt und können dann ohne weiteres aus einem Niederschlagsammler entnommen und durch ein neues Speicherglied ersetzt werden, wobei die Auswertung dann im Labor erfolgen kann.

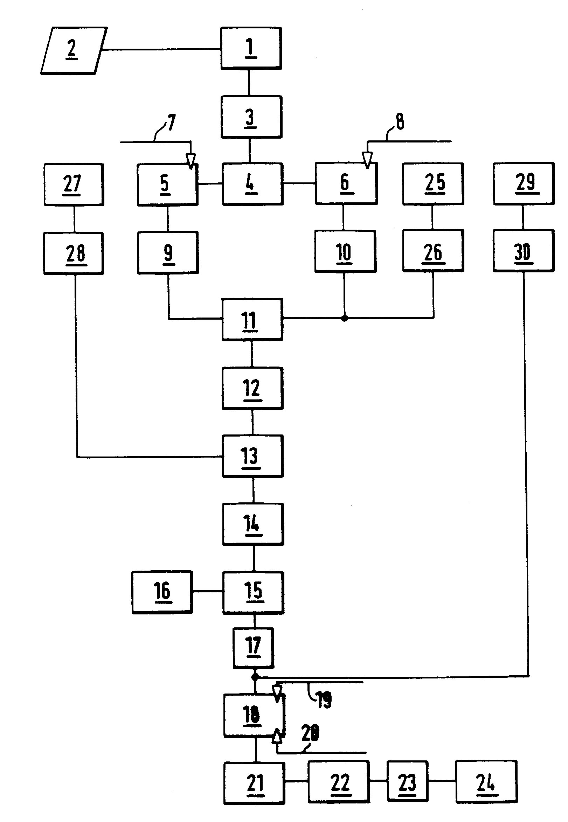

In der Zeichnung ist die Erfindung an Hand eines Niederschlagsammlers veranschaulicht, dessen einzelne Teile durch ein Blockschaltbild symbolisiert sind.

Der Niederschlagsammler weist in an sich bekannter Weise einen Sammeltrichter (1) auf, der - was hier nicht näher dargestellt ist - mit einem Abdeckmechanismus, beispielsweise mit einem elektromotorisch betätigbaren Deckel, versehen ist. Entsprechende Konstruktionen sind allgemein bekannt, beispielsweise durch die VDI-Richtlinien 3870 vom Juli 1985. Der Abdeckmechanismus wird durch einen Niederschlagsfühler (2) gesteuert, bei dessen Benetzung mit Regen, Schnee, Tau oder dergleichen ein Impuls an den Abdeckmechanismus gegeben wird, damit der Deckel von dem Sammeltrichter (1) entfernt wird.

Der Niederschlagsfühler (2) kann zusätzlich mit einer Heizeinrichtung versehen sein, um Niederschlag in Form von Schnee oder Reif in die flüssige Form überzuführen.

Unterhalb des Sammeltrichters (1) ist ein Filter (3) angeordnet, der im Wasser enthaltene feste Bestandteile abfiltert. Das Wasser gelangt dann zu einem Dreiwegeventil (4), über den es je nach seiner Stellung in die erste Volumenmeßzelle (5) oder in die zweite Volumenmeßzelle (6) geleitet wird. Die Volumenmeßzellen (5, 6) sind jeweils mit einem Maximumsensor (7, 8) versehen, die über eine Steuereinrichtung auf das Dreiwegeventil (4) derart wirken, daß es bei Ansprechen eines Maximumsensors (7 bzw 8) auf die jeweils andere Volumenmeßzelle (6 bzw. 5) umschaltet. An beide Volumenmeßzellen (5, 6) schließen sich jeweils ein Steuerventil (9, 10) an, deren Ausgänge in eine Leitfähigkeitsmeßzelle (11) münden. Derartige Leitfähigkeitsmeßzellen (11) sind an sich bekannt, so daß sie keiner näheren Beschreibung bedürfen.

Der Ausgang der Leitfähigkeitsmeßzelle (11) wird durch ein weiteres Steuerventil (12) gesteuert, an das sich eine ph-Wert-Meßzelle (13) anschließt. Auch solche Meßzellen sind bei Geräten der vorliegenden Art an sich bekannt.

In Strömungsrichtung des Wassers gesehen hinter der ph-Wert-Meßzelle (13) sind ein weiteres Steuerventil (14) und ein Dreiwegeventil (15) angeordnet. Ein Ausgang des Dreiwegeventils (15) führt zu einem Abwasserbehälter (16), während der andere Ausgang zu einer Pumpe (17) geht, über die das Wasser zu einem Sammelbehälter (18) gefördert wird. Der Sammelbehälter (18) weist einen Maximumsensor (19) und einen Minimumsensor (20) auf. Hinter dem Sammelbehälter (18) ist eine Adsorptionssäule (21) angeordnet, die dazu bestimmt ist, organische Schadstoffe aus dem zu untersuchenden Wasser herauszuholen und sie dann anschließend einer Analyse zuzuführen. Es kann sich beispielsweise um eine mit einem polymeren Adsorberharz gefüllte Glassäule handeln wie es z.B. von der Fa. SERVA Feinbiochemika, D-6900 Heidelberg 1, unter dem Warenzeichen "Amberlite" vertrieben wird. Zum Passieren der Adsorptionssäule (21) wird das Wasser nach Öffnen eines Steuerventils (22) durch eine nachgeschaltete Pumpe (23) aus dem Sammelbehälter (20) herausgesaugt, passiert dann die Adsorptionssäule (21) und gelangt anschließend in einen weiteren Sammelbehälter (24).

Die Leitfähigkeitsmeßzelle (11) hat auch Verbindung zu einem Vorratsbehälter (25), in dem destilliertes Wasser gespeichert wird, das über ein Steuerventil (26) in die Leitfähigkeitsmeßzelle (11) geleitet werden kann. Die ph-Wert-Meßzelle (13) ist mit einem weiteren Vorratsbehälter (27) über ein Steuerventil (28) verbunden, wobei der Vorratsbehälter (27) eine Kalibrierlösung enthält. Schließlich ist ein dritter Vorratsbehälter (29) vorhanden, dessen Ausgang über ein Steuerventil (30) zum Sammelbehälter (18) führt.

Zur Steuerung der einzelnen Komponenten des vorbeschriebenen Niederschlagsammlers ist eine elektronische Steuereinrichtung vorgesehen, die mit einem Einplatinencomputer mit einem Siemens Mikroprozessor SAB 80535 bestückt ist. Die Steuereinrichtung wird durch ein Powernetzteil gespeist.

Nach dem Einschalten wird der Niederschlagssammler zunächst in einen Standby-Zustand versetzt. Hierzu werden die Steuerventile (9, 10, 12, 14, 22, 26, 28, 30) in Ausgangsstellung gebracht, d.h. geschlossen. Das Dreiwegeventil (4) wird auf die erste Volumenmeßzelle (5) und das Dreiwegeventil (15) auf den Abwasserbehälter (16) eingestellt. Gleichzeitig werden Clock und Timer initialisiert. Dann wird das Steuerventil (26) geöffnet, so daß destilliertes Wasser aus dem Vorratsbehälter (25) in die Leitfähigkeitsmeßzelle (11) und anschließend in die ph-Wert-Meßzelle (13) gelangt. Dann wird das Steuerventil (28) geöffnet, so daß die ph-Wert-Meßzelle (13) mit Kalibrierlösung aus dem Vorratsbehälter (27) gefüllt wird. Es erfolgt dann ein Autotest der beiden Meßzellen (11, 13) mit anschließender Initialisierung. Der Niederschlagssammler ist dann im Standby-Zustand.

Im Standby-Betrieb werden ständig der Niederschlagsfühler (2) sowie der im Sammelbehälter (18) angeordnete Minimumsensor (20) abgefragt. Stellt sich bei der Abfrage heraus, daß der Minimumsensor (20) nicht mehr benetzt ist, so öffnet die Steuereinrichtung das Steuerventil (30), so daß in den Sammelbehälter (18) destilliertes Wasser aus dem Vorratsbehälter (29) fließt, das anschließend in die Adsorptionssäule (21) gelangt. Auf diese Weise wird die Adsorptionssäule (21) vor der Zerstörung bewahrt.

Setzt nun Niederschlag ein, wird dies von dem Niederschlagsfühler (2) erfaßt. Er gibt einen Impuls an die Steuereinrichtung, so daß deren Deckel geöffnet wird. Gleichzeitig werden Datum und Uhrzeit und damit der zeitliche Beginn des Regenereignisses abgespeichert. Ferner werden die Steuerventile (26, 12 und 14) geöffnet, so daß destilliertes Wasser aus dem Vorratsbehälter (25) durch die Leitfähigkeitsmeßzelle (11) und die ph-Wert-Meßzelle (13) fließt. Zuvor wird in letzterer die am Ende einer vorangegangenen Messung zurückgehaltene Meßprobe in den Sammelbehälter (18) abgesaugt. Handelt es sich um das erste Regenereignis nach dem Einschalten, wird die dann noch in der ph-Wert-Meßzelle vorhandene Kalibrierlösung mit dem destillierten Wasser herausgewaschen. Über das Dreiwegeventil (15) gelangen das destillierte Wasser und gegebenenfalls Kalibrierlösung in den Abwasserbehälter (14). Nach dem Öffnen des Deckels des Sammeltrichters (1) wird das Spülen der Leitfähigkeits- und der ph-Wert-Meßzellen (11, 13) durch Schließen der Steuerventile (26, 12 und 14) beendet.

Anschließend wird abgefragt, ob die erste Volumenmeßzelle (5) gefüllt ist. Ist dies der Fall, wird das Wasser nach Öffnen des Steuerventils (9) in die Leitfähigkeitsmeßzelle (11) geleitet, während frische Kalibrierlösung aus dem Vorratsbehälter (27) in die ph-Wert-Meßzelle (13) gefüllt wird.

Ist die erste Volumenmeßzelle (5) noch nicht gefüllt, wird zunächst nochmals das Steuerventil (26) betätigt, so daß die Leitfähigeitsmeßzelle (11) wieder mit destilliertem Wasser gefüllt wird. Entsprechendes geschieht mit dem Steuerventil (24), so daß frische Kalibrierlösung in die ph-Wert-Meßzelle (11) gelangt. Erreicht nun das Wasser in der ersten Volumenmeßzelle (5) den Maximumsensor (7), wird das Dreiwegeventil (4) auf die zweite Volumenmeßzelle (6) umgeschaltet, so daß das nachfließende Wasser in dieser Volumenmeßzelle (6) gesammelt wird. Gleichzeitig wird das Steuerventil (9) geöffnet, so daß das in der ersten Volumenmeßzelle (5) gesammelte Wasser in die Leitfähigkeitsmeßzelle (11) fließen kann. Datum, Uhrzeit und Nummer der Füllung werden zur selben Zeit abgespeichert. Bevor das Wasser in die Leitfähigkeitsmeßzelle (11) einläuft, werden diese wie auch die ph-Wert-Meßzelle (13) in den Abwasserbehälter (16) entleert. Die ph-Wert-Meßzelle (13) wird dann sofort erneut mit einer Kalibrierlösung gefüllt, nachdem die Steuerventile (12) und (14) wieder geschlossen worden sind.

Das aus der ersten Volumenmeßzelle (5) kommende Wasser wird nun auf seine Leitfähigkeit analysiert. Hierfür werden so lange Werte abgefragt, bis diese stabil sind oder die zur Verfügung stehende Meßzeit abgelaufen ist. Der Mittelwert wird anschließend mit der Uhrzeit abgespeichert. Danach wird die ph-Wert-Meßzelle (13) durch Öffnen des Steuerventils (14) zum Abwasserbehälter (16) entleert und durch Ansteuerung des Steuerventils (12) mit dem aus der Leitfähigkeitsmeßzelle (11) kommenden Wasser gefüllt. Gleichzeitig schließt das Steuerventil (14) und wird das Dreiwegeventil (15) in Richtung auf den Sammelbehälter umgeschaltet. Dann wird der ph-Wert gemessen und nach Erreichen eines stabilen Wertes mit der Uhrzeit abgespeichert.

Die ph-Wert-Messung und die Leitfähigkeitsmessung dürfen zusammen nicht länger als drei Minuten dauern. Ist innerhalb dieser Zeit kein stabiler Wert erreicht, wird der letzte Meßwert mit einem Hinweis auf die Instabilität abgespeichert.

Mit dem Verlassen des Wassers aus der Leitfähigkeitsmeßzelle (11) wird durch die Steuereinrichtung geprüft, ob die zweite Volumenmeßzelle (6) schon voll ist. Ist dies nicht der Fall, wird durch Ansteuerung des Steuerventils (26) wieder destilliertes Wasser in die Leitfähigkeitsmeßzelle (11) gefüllt, damit diese nicht austrocknet. Ist die zweite Volumenmeßzelle (6) schon voll und hat deshalb auch schon der Maximumsensor (8) zwecks Umstellung des Dreiwegeventils (4) angesprochen, wird das in ihr gesammelte Wasser durch Betätigung des Steuerventils (10) sofort in die Leitfähigkeitsmeßzelle (11) geleitet, wo es so lange bleibt, bis das Wasser aus der ersten Volumenmeßzelle (5) die ph-Wert-Meßzelle (13) verläßt. Das Wasser in der ph-Wert-Meßzelle (13) wird dann über die Pumpe (17) in den Sammelbehälter (18) abgesaugt.

Durch Wiederholung der vorgenannten Abfolge füllt sich der Sammelbehälter (18) so lange, bis der Maximumsensor (19) anspricht. Dieser setzt nach Öffnung des Steuerventils (22) die Pumpe (23) in Betrieb, so daß das im Sammelbehälter (18) vorhandene Wasser durch die Adsorptionssäule (21) in den entgültigen Sammelbehälter (24) gesaugt wird. In der Adsorptionssäule (21) wird das Wasser von organischen Schadstoffen befreit, so daß es nach Überführung in den Sammelbehälter (24) für die Schwermetallanalyse geeignet ist.

Ist der Sammelbehälter (18) bis über den Maximumsensor (19) gefüllt, so wird er während des nächsten Meßvorgangs mit der Pumpe (19) über die Adsorptionssäule (17) bis zum Ansprechen des Minimumsensors (27) leergepumpt. Anschließend wird das Steuerventil (22) wieder geschlossen.

Das Ende eines Regenereignisses wird von dem Niederschlagsfühler (2) erfaßt, weil er dann nicht mehr benetzt ist. Durch ein Zeitglied wird jetzt noch zwei Minuten abgewartet. Fällt in dieser Zeit kein Regen mehr, wird der Deckel des Sammeltrichters (1) geschlossen und der Niederschlagssammler geht wieder in den Standby-Betrieb über. Das letzte Wasser bleibt in der ph-Wert-Meßzelle (11) zurück, während die Leitfähigkeitsmeßzelle (11) mit destilliertem Wasser aus dem Vorratsbehälter (25) gefüllt wird.

## Patentansprüche

1. Verfahren zur Analyse von Wasser oder anderen Flüssigkeiten, insbesondere von Niederschlagswasser in Regensammlern, bei dem das Wasser für eine Mengenmessung zunächst in einer Volumenmeßzelle (5, 6) bis zum Erreichen eines bestimmten, immer gleichen Füllstandes gesammelt wird, bevor das so gesammelte Wasser jeweils an eine nachfolgende Eigenschaftsmeßzelle (11, 13), insbesondere einer Leitfähigkeitsmeßzelle und/oder einer pH-Wert-Meßzelle, weitergegeben wird,
dadurch gekennzeichnet, daß das Wasser abwechselnd in zumindest zwei parallelen Volumenmeßzellen (5, 6) gesammelt und abwechselnd an die nachfolgende Eigenschaftsmeßzelle (11) weitergegeben wird.

2. Verfahren nach Anspruch 1,
dadurch gekennzeichnet, daß das Wasser durch eine Adsorptionssäule (21) zur Entfernung organischer Bestandteile geleitet und anschließend gesammelt wird.

3. Verfahren nach Anspruch 2,
dadurch gekennzeichnet, daß das Wasser zuvor in einem Sammelbehälter (18) gesammelt und dann bis auf einen bestimmten Rest durch die Adsorptionssäule (21) geleitet wird.

4. Verfahren nach Anspruch 3,
dadurch gekennzeichnet, daß die Adsorptionssäule (21) mit destilliertem Wasser gefüllt wird, wenn das Restwasser im Sammelbehälter (18) unter ein bestimmtes Niveau absinkt.

5. Verfahren nach Anspruch 3 oder 4,
dadurch gekennzeichnet, daß das Wasser erst bei Erreichen eines bestimmten Maximalstandes durch die Adsorptionssäule (21) oder am Ende einer Messung geleitet wird.

6. Verfahren nach einem der Ansprüche 2 bis 5,
dadurch gekennzeichnet, daß das Wasser durch die Adsorptionssäule (21) gesaugt wird.

7. Vorrichtung zur Durchführung des Verfahrens nach einem der Ansprüche 1 bis 6, mit einer Mengenmeßeinrichtung und zumindest einer nachfolgenden Eigenschaftsmeßzelle (11), insbesondere einer Leitfähigkeitsmeßzelle und/oder einer pH-Wert-Meßzelle, wobei die Mengenmeßeinrichtung eine mit einer Füllstandssonde (7, 8) ausgestatteten Volumenmeßzelle (6) aufweist, die derart ansteuerbar ist, daß das Wasser erst bei Erreichen der Füllstandssonde (7, 8) aus der jeweiligen Volumenmeßzelle (5, 6) herausgelassen und zur Eigenschaftsmeßzelle weitergeleitet wird,
dadurch gekennzeichnet, daß wenigstens zwei Volumenmeßzellen (5, 6) vorgesehen sind, die abwechselnd durch eine Steuereinrichtung derart ansteuerbar sind, daß der Zufluß nur zu jeweils einer Volumenmeßzelle (5, 6) geöffnet ist und bei Erreichen der Füllstandssonde (7, 8) in dieser Volumenmeßzelle (5, 6) geschlossen und zu einer anderen Volumenmeßzelle (5, 6) geöffnet wird.

8. Vorrichtung nach Anspruch 7,
dadurch gekennzeichnet, daß eine Adsorptionssäule (21) zur Entfernung organischer Bestandteile vorgesehen und daran anschließend ein weiterer Sammelbehälter (24) nachgeschaltet sind.

9. Vorrichtung nach Anspruch 8,
dadurch gekennzeichnet, daß vor der Adsorptionssäule (21) ein weiterer Sammelbehälter (18) angeordnet ist.

10. Vorrichtung nach Anspruch 9,
dadurch gekennzeichnet, daß der weitere Sammelbehälter (18) mit einem mit der Steuereinrichtung in Verbindung stehenden Füllstandssensor (20̸) für die Ermittlung eines Minimalniveaus ausgestattet ist und daß die Adsorptionssäule (21) mit einem Vorratsbehälter (29) für destilliertes Wasser (30̸) verbunden ist, deren Zulauf durch die Steuereinrichtung geöffnet wird, wenn der Füllstandssensor (20̸) ein Unterschreiten des Minimalniveaus meldet.

11. Vorrichtung nach Anspruch 9 oder 10̸,
dadurch gekennzeichnet, daß der weitere Sammelbehälter (18) mit einem Füllstandssensor (19) zu Erfassung eines Maximalniveaus ausgestattet ist, der über die Steuereinrichtung einer Pumpe (22) zur Förderung des Wassers durch die Adsorptionssäule (21) derart verbunden ist, daß die Pumpe (22) bei Erreichen des Maximalniveaus in Betrieb gesetzt wird.

12. Vorrichtung nach einem der Ansprüche 9 bis 11,
dadurch gekennzeichnet, daß zwischen einer Eigenschaftsmeßzelle (13) und dem weiteren Sammelbehälter (18) ein Dreiwegeventil (15) zur Freigabe einer Leitung zu einem Abwasserbehälter (16) vorgesehen ist.

13. Vorrichtung nach einem der Ansprüche 7 bis 12,
dadurch gekennzeichnet, daß die Vorrichtung als Niederschlagssammler mit einem Regenfühler (2) ausgebildet ist, wobei der Regenfühler (2) Beginn und Ende einer Messung bestimmt.

14. Vorrichtung nach Anspruch 13,
dadurch gekennzeichnet, daß der Regenfühler (2) mit einem Verzögerungsglied zur Beendigung der Messung in einem zeitlichen Abstand nach Wegfall der Benetzung versehen ist.

15. Vorrichtung nach einem der Ansprüche 7 bis 14,
dadurch gekennzeichnet, daß die Vorrichtung ein herausnehmbares Speicherglied für die Speicherung von Daten aufweist, insbesondere von Datum und Uhrzeit der jeweils ersten Benetzung des Regenfühlers (2), von Datum, Uhrzeit und Nummer der Füllung einer Volumenmeßzelle (5, 6), der Gesamtniederschlagsmenge sowie gegebenenfalls der Leitfähigkeit und des pH-Wertes und schließlich von Datum und Uhrzeit des Meßendes.

## Claims

1. A method for analyzing water or other liquids, in particular precipitacion water in rain collectors, wherein the water for a quantitative measurement first is collected in a volumetric cell (5, 6) until a specified, always the same filling level has been reached, before the water collected in this manner is fed to a subsequent property-determining cell (11, 13), in particular in a conductivity-determining cell and/or a pH-determining cell,
characterized in that the water is collected alternatingly in at least two parallel volumetric cells (5, 6) and is alternatingly fed to the following property-determining cell (11).

2. Method defined in Claim 1,
characterized in that the water is made to pass through an adsorption columm (21) to remove organic components and thereupon is collected.

3. Method defined in Claim 2,
characterized in that the water is previously collected in a collecting vessel (18) and then is made to pass down to a specified remainder through the adsorption column (21).

4. Method defined in Claim 3,
characterized in that the adsorption column (21) will be filled with distilled water when the remaining water in the collecting vessel (18) becomes less than a given level.

5. Method defined by either of Claims 3 and 4,
characterized in that the water will be made to pass through the adsorption column (21) only after a specified maximum level has been reached or at the end of a measurement.

6. Method defined by one of Claims 2 through 5,
characterized in that the water is aspirated through the adsorption column (21).

7. Apparatus to carry out the method defined by one of Claims 1 through 6, comprising a quantitative device and at least one subsequent property-determining cell (11), in particular a conductivity-determining cell and/or a pH-determining cell, the quantitative device comprising a volumetric cell (6) equipped with a filled-level sensor (7, 8), where said cell is driven in such a manner that the water shall be discharged from the particular volumetric cell (5, 6) and moved to the property-determining cell only upon its reaching the filled-level sensor (7, 8),
characterized in that at least two volumetric cells (5, 6) are provided, which are alternatingly driven by a control device in such a manner that the feed to only one volumetric cell (5, 6) is opened at a time and when the filled-level sensor (7,8) in this volumetric cell (5, 6) is reached, it will be closed and the feed to another volumetric cell (5, 6) will be opened.

8. Apparatus defined in Claim 7,
characterized in that an adsorption column (21) is provided to remove organic components and a further collecting vessel (24) is connected at the outlet side thereto.

9. Apparatus defined in Claim 8,
characterized by a further collecting vessel (18) preceeding the adsorption column (21).

10. Apparatus defined in Claim 9,
characterized in that the further collecting vessel (18) is equipped with a level sensor (20̸) communicating with the control device to detect a minimum level and in that the adsorption column (21) communicates with a reservoir (29) for distilled water (30̸), the intake thereof being opened by the control device when the level sensor (20̸) transmits a signal the minimum level has been crossed downward.

11. Apparatus defined by Claim 9 or 10̸,
characterized in that the further collecting vessel (18) is equipped with a filled-state sensor (19) to detect a maximum level and so connected by means of the control device of a pump (22) moving the water through the adsorption column (21) that the pump (22) becomes operative when the maximum level is reached.

12. Apparatus defined by one of Claims 9 through 11,
characterized in that a three-way valve (15) releasing a conduit to a drainage container (16) is provided between a property-determining cell (13) and the further collecting vessel (18).

13. Apparatus defined by one of Claims 7 through 12,
characterized in that the apparatus is designed as a precipitation collector with a rain sensor (2) determining the beginning and the end of the measurement.

14. Apparatus defined in Claim 13,
characterized in that the rain sensor (2) is equipped with a time-delay means to terminate the measurement at a time interval following the end of wetting.

15. Apparatus defined by one of Claims 7 through 14,
characterized by this apparatus comprising an removeable memory unit to store data, in particular the time and date of the particular first wetting of the rain sensor (2), the date, time and filling number of a volumetric cell (5, 6), the total amount of precipitation and , should the occasion arise, for the conductivity and pH-value and lastly the time and date of end of measurement.

## Revendications

1. Procédé d'analyse de l'eau ou d'autres liquides, notamment d'eau de pluie dans des collecteurs d'eau de pluie, qui consiste à recueillir l'eau pour une mesure de quantité d'abord dans une cellule de mesure du volume (5,6) jusqu'à obtention d'un niveau déterminé toujours identique, avant d'envoyer l'eau ainsi recueillie à une cellule de mesure d'une propriété (11,13) en aval, notamment à une cellule de mesure de la conductivité et/ou à une cellule de mesure du pH,
caractérisé en ce qu'il consiste à recueillir l'eau alternativement dans au moins deux cellules de mesure de volume (5, 6) en parallèle et à l'envoyer alternativement à la cellule de mesure d'une propriété (11) en aval.

2. Procédé suivant la revendication 1,
caractérisé en ce qu'il consiste à faire passer l'eau dans une colonne d'adsorption (21) pour éliminer des constituants organiques et ensuite à la recueillir.

3. Procédé suivant la revendication 2,
caractérisé en ce qu'il consiste à recueillir l'eau d'abord dans une cuve collectrice (18) et à la faire passer ensuite dans la colonne d'adsorption (21), à l'exception d'un résidu déterminé.

4. Procédé suivant la revendication 3,
caractérisé en ce qu'il consiste à emplir la colonne d'adsorption (21) d'eau distillée si l'eau résiduelle dans la cuve collectrice (18) s'abaisse en dessous d'un niveau déterminé.

5. Procédé suivant la revendication 3 ou 4,
caractérisé en ce qu'il consiste à ne faire passer l'eau dans la colonne d'adsorption (21) que lorsqu'un niveau maximum déterminé est atteint ou à la fin d'une mesure.

6. Procédé suivant l'une des revendications 2 à 5,
caractérisé en ce qu'il consiste à aspirer l'eau dans la colonne d'adsorption (21).

7. Appareil pour la mise en oeuvre du procédé suivant l'une des revendications 1 à 6 comprenant un dispositif de mesure de la quantité et au moins une cellule de mesure d'une propriété (11) en aval, notamment une cellule de mesure de la conductivité et/ou une cellule de mesure du pH, le dispositif de mesure de la quantité comportant une cellule de mesure du volume (6) munie d'une sonde de niveau (7, 8) et pouvant être commandée de façon que l'eau ne sorte de la cellule de mesure du volume (5, 6) et ne soit envoyée à la cellule de mesure d'une propriété que lorsqu'est atteinte la sonde de niveau (7, 8),
caractérisé en ce qu'il est prévu au moins deux cellules de mesure de volume (5, 6) qui peuvent être commandées alternativement par un dispositif de commande, de manière que l'accès ne soit donné qu'à l'une des cellules de mesure de volume (5, 6) et que, lorsque la sonde de niveau (7, 8) est atteinte dans cette cellule du mesure de volume (5, 6), l'accès à celle-ci soit interdit et l'accès soit donné à une autre cellule du mesure de volume (5, 6).

8. Appareil suivant la revendication 7,
caractérisé par une colonne d'adsorption (21) pour éliminer les constituants organiques et par une autre cuve collectrice (24) en aval.

9. Appareil suivant la revendication 8,
caractérisé par une autre cuve collectrice (18) en amont de la colonne d'adsorption (21).

10. Appareil suivant la revendication 9,
caractérisé en ce que l'autre cuve collectrice (18) est munie d'un détecteur de niveau (20) relié au dispositif de commande et destiné à déterminer un niveau minimum et en ce que la colonne d'adsorption (21) communique avec un réservoir (29) d'eau distillée (30), dont l'accès est ouvert par le dispositif de commande quand le détecteur de niveau (20) indique que l'on est passé en-dessous du niveau minimum.

11. Appareil suivant la revendication 9 ou 10,
caractérisé en ce que l'autre cuve collectrice (18) est munie d'un détecteur de niveau (19) destiné à détecter un niveau maximum, qui est relié par le dispositif de commande d'une pompe (22) destinée à véhiculer de l'eau dans la colonne d'adsorption (21), de manière à mettre la pompe (22) en fonctionnement lorsque le niveau maximum est atteint.

12. Appareil suivant l'une des revendications 9 à 11,
caractérisé en ce qu'il est prévu, entre une cellule de mesure d'une propriété (13) et l'autre cuve collectrice (18), une vanne (15) à trois voies pour dégager un conduit menant à une cuve (16) d'eau résiduaire.

13. Appareil suivant l'une des revendications 7 à 12,
caractérisé en ce que l'appareil est constitué en collecteur d'eau de pluie ayant une sonde (2) de pluie, la sonde (2) de pluie déterminant le début et la fin d'une mesure.

14. Appareil suivant la revendication 13,
caractérisé en ce que la sonde (2) de pluie est munie d'un élément temporisateur pour mettre fin à la mesure dans un laps de temps donné après qu'a cessé le mouillage.

15. Appareil suivant l'une des revendications 7 à 14,
caractérisé en ce que l'appareil comporte un élément de mémorisation amovible pour mémoriser des données, notamment la date et l'heure du premier mouillage de la sonde de pluie (2), la date, l'heure et le rang de remplissage d'une cellule de mesure de volume (5, 6), la quantité totale de précipitations ainsi que, le cas échéant, la conductivité et le pH et enfin, la date et l'heure de la fin de la mesure.
